# EUROPÄISCHE PATENTANMELDUNG

(11) **EP 2 050 812 A1**
(43) Veröffentlichungstag der Anmeldung: **22.04.2009**
(21) Anmeldenummer: 07020398.9
(22) Anmeldetag: 18.10.2007
(51) Int. Cl.: C12M 1/107, C12M 1/36

(54) **Biogasanlage**

(71) Anmelder: Bayval, Kadri, 29494 Trebel (DE)
(72) Erfinder: Bayval, Kadri, 29494 Trebel (DE)
(74) Vertreter: Walkenhorst, Andreas

(57) **Zusammenfassung**

Ein Verfahren zum Betreiben einer Biogasanlage (1), bei dem in einer Gaserzeugungskammer (2) eine Anzahl von Messwerten erfasst und überwacht werden, wobei zur Prozessführung eine Anzahl von Stellwerten an die Gaserzeugungskammer (2) übertragen werden soll eine effizientere Gasausbeute und damit eine bessere Wirtschaftlichkeit ermöglichen. Dazu werden die Stellwerte in Abhängigkeit von vorgegebenen Sollwerten zur Regelung der Gasausbeute als Führungsgröße ermittelt.

## Beschreibung

Die Erfindung bezieht sich auf ein Verfahren zum Betreiben einer Biogasanlage, bei dem in einer Gaserzeugungskammer eine Anzahl von Messwerten erfasst und überwacht werden, wobei zur Prozessführung eine Anzahl von Stellwerten an die Gaserzeugungskammer übertragen werden. Sie betrifft weiterhin ein Verfahren zum Betreiben eines Anlagenverbundes mit einer Mehrzahl von Biogasanlagen sowie eine Biogasanlage und einen Anlagenverbund von Biogasanlagen.

Das Funktionsprinzip einer Biogasanlage beruht dabei auf einem biologischen Prozess. Bei der Gärung einer organischen Masse entsteht unter Ausschluss von Sauerstoff ein Gasgemisch, welches aus etwa 2/3 Methan, 1/3 Kohlendioxid und Restmengen von Wasserstoff, Schwefelwasserstoff, Ammoniak und anderen Gasen zusammengesetzt ist. Dieser in der Natur verbreitete Prozess findet beispielsweise in Mohren, auf dem Grund von Seen, in der Güllegrube sowie im Pansen von Wiederkäuern statt. Hierbei wird die organische Masse fast vollständig in Biogas umgewandelt und nur geringe Restmengen an neuer Biomasse bleiben übrig.

In Deutschland werden aktuell ca. 3500 Biogasanlagen unterschiedlicher Größe betrieben. In ihnen wird das durch die Biomasse gewonnene Biogas hauptsächlich mit Hilfe von Verbrennungsmotoren in elektrische Energie umgewandelt. Vereinzelt wird das Biogas mit Mikrogasturbinen, Brennstoffzellen oder Stirlingmotoren in elektrische Energie umgewandelt. In einzelnen Biogasanlagen wird das Biogas ausschließlich thermisch verwertet oder als Bioautogas an die Verbraucher abgegeben.

Im Gegensatz zu chemischen Prozessen lassen sich biologische Prozesse nicht präzise steuern. Es gibt jedoch Beispiele in der industriellen Produktion mit dominierten biologischen Prozessen, wie z. B. in Großbäckereien, dass diese zur Erreichung einer gleich bleibenden Qualität geregelt werden können. Ein solches standardisiertes System ist allerdings auf Biogasanlagen nicht übertragbar. Zum einen variieren die Systemparameter, wie beispielsweise die Größe, die Umgebungstemperatur, die Luftfeuchtigkeit, stark von Anlage zu Anlage, zum anderen werden aber auch die Biogasanlagen mit unterschiedlichen Biomassen gefüttert. Dies führt dazu, dass keine allgemeingültigen Parameter bzw. Einstellungen für Biogasanlagen existieren und diese daher aufgrund mangelnder Kenntnis im Rahmen ihrer Möglichkeiten nicht effizient genug arbeiten.

Der Erfindung liegt daher die Aufgabe zugrunde, ein Verfahren zum Betreiben einer Biogasanlage anzugeben, die eine effizientere Gasausbeute und damit eine bessere Wrtschaftlichkeit möglich. Des Weiteren soll eine zur Durchführung des Verfahrens besonders geeignete Biogasanlage angegeben werden.

Bezüglich der Vorrichtung wird diese Aufgabe erfindungsgemäß gelöst, indem die Stellwerte in Abhängigkeit von vorgegebenen Sollwerten zur Regelung der Gasausbeute als Führungsgröße ermittelt werden.

Die Erfindung geht von der Überlegung aus, dass ein effizienter Betrieb einer Biogasanlage anhand der im Verhältnis zur verwendeten Biomasse als Futtermittel erreichbaren Gasausbeute bestimmt werden kann. Zur Optimierung der Gasausbeute sollten daher für die Gaserzeugung charakteristische Größen wie Futtermenge, Temperatur und/oder pH-Wert berücksichtigt und gegebenenfalls an vorgegebene Erfahrungswerte angepasst werden. Diese charakteristischen Werte sollten daher im Betrieb der Biogasanlage mittels Sensoren bestimmt, überwacht und ausgewertet werden. Eine gemessene Abweichung der Messwerte von den vorgegebenen Sollwerten sollte daher von dem System erkannt und die Biogasanlage zur Verbesserung der Gasausbeute neu eingestellt werden. Dazu werden aus den Messwerten und vorgegebenen Sollwerten Stellwerte ermittelt, die zur Regelung der Gasausbeute an die Gaserzeugungskammer übertragen werden.

Vorteilhafterweise wird dabei für eine besonders hohe Anlageneffizienz die Ansteuerung der Gaserzeugungskammer unter Berücksichtigung der im Hinblick auf die zugeführte Biomasse theoretisch maximal möglichen Gasausbeute vorgenommen. Dazu wird die zugeführte Biomasse oder der Futterstrom zweckmäßigerweise in der Art einer Voranalyse stichprobenartig untersucht, wobei anhand von entnommenen Proben die theoretisch erreichbare Gasausbeute bestimmt wird. Diese wird dann vorteilhafterweise bei der Vorgabe der Sollwerte berücksichtigt.

Zur Protokollierung, aber auch zur Ansammlung eines Datenbankgrundstockes werden in Abhängigkeit der Kapazität in vorteilhafter Ausführung in regelmäßigen Abständen Messwerte und der aus ihnen ermittelte Ist-Wert für die Gasausbeute in einer Datenbank hinterlegt. In zusätzlicher vorteilhafter Ausführung werden zusammen mit den Messwerten und dem Ist-Wert für die Gasausbeute auch die Systemparameter wie beispielsweise die Größe oder Umgebungsparameter der Biogasanlage in der Datenbank hinterlegt. Dadurch wird eine Art Betriebsprotokoll der Biogasanlage generiert, welches für zukünftige Weiterentwicklungen als Basis für eine verbesserte Gasausbeute dienen kann.

In besonders vorteilhafter Ausführung werden die Messwerte, die aus ihnen ermittelten Ist-Werte der Gasausbeute und die Systemparameter von einer Mehrzahl von Biogasanlagen in einer gemeinsamen zentralen Datenbank hinterlegt. Hierdurch ist eine Vergleichsmöglichkeit bzgl. unterschiedlicher Bedingungen wie beispielsweise Größe, Umgebungsvariablen und/oder Biomassen gegeben.

In besonders vorteilhafter Ausführung werden die Sollwerte über eine trainierbare Schätzeinheit oder ein neuronales Netz eingelernt. Damit wird durch die Verwendung der trainierbaren Schätzeinheit zur Ermittlung der Gasausbeute eine besonders bedarfs- und zustandsgerechte Auswertung des aktuellen Systemverhaltens und eine dementsprechend angepasste Ausgabe von Meldungen allein aufgrund einer beobachteten Änderung des Systemverhaltens möglich, ohne dass hierzu direkt oder mittelbar die eigentliche Gasausbeute einer Biogasanlage als Kenngröße ermittelt oder berücksichtigt werden müsste. Dies führt im Umkehrschluss dazu, dass es durch die trainierbare Schätzeinheit und die Sammlung der Messdaten und Systemparameter von verschiedenen Biogasanlagen in einer gemeinsamen Datenbank möglich ist, Sollwerte für einen besonders effizienten Betrieb einer Biogasanlage unter gegebenen Systemparametern bereitzustellen.

Daher werden in vorteilhafter Ausführung beispielsweise bei der Neuinstallation einer Biogasanlage oder in regelmäßigen Abständen neue verbesserte Sollwerte von der Biogasanlage bei der trainierbaren Schätzeinheit abgerufen und durch diese der Betrieb der Biogasanlage optimiert. In alternativer oder zusätzlicher vorteilhafter Ausführung kann die Übertragung der Sollwerte auch durch Befehl seitens der trainierbaren Schätzeinheit, beispielsweise in Form von regelmäßigen Updates, ausgelöst werden. Hierdurch können die Biogasanlagen zentral zum einen Überwacht, zum anderen auch für eine optimale Effizienz ferngesteuert werden. Die Genauigkeit und Zuverlässigkeit der Sollwerte steigt dabei mit steigendem Dateninhalt in der gemeinsamen zentralen Datenbank.

Bezüglich der Biogasanlage wird die genannte Aufgabe gelöst, indem eine Reglereinheit in Abhängigkeit der an diese übermittelten Messwerte und vorgegebenen Sollwerte zur Regelung der Gasausbeute ausgelegt ist. In vorteilhafter Ausführung werden in einer solchen Biogasanlage die Messwerte, der für diese Messwerte ermittelte Ist-Wert der Gasausbeute und/oder die Systemparameter hinterlegt.

In besonders vorteilhafter Ausführung werden diese Daten von einer Mehrzahl von Biogasanlagen in einer gemeinsamen zentralen Datenbank hinterlegt. Dabei ist in vorteilhafter Ausführung eine an der zentralen Datenbank angeschlossene und eine trainierbare Schätzeinheit umfassende Einstelleinheit zur Fernregelung mindestens einer Biogasanlage ausgelegt.

Die mit der Erfindung erzielten Vorteile bestehen insbesondere darin, dass durch die Bestimmung von Stellwerten aufgrund eines Soll-Ist-Wert-Vergleiches zur Regelung der Gasausbeute als Führungsgröße ein effizienter und zuverlässiger Betrieb der Biogasanlage erreichbar ist. Die Speicherung der Messwerte, der aus ihnen ermittelten Ist-Werte für die Gasausbeute und der Systemparameter in einer gemeinsamen Datenbank durch eine Mehrzahl von Biogasanlagen ist es möglich, eine trainierbare Schätzeinheit zu füttern, mit deren Hilfe eine optimale Regelung und damit effiziente Nutzung von Biogasanlagen möglich wird. Weiterhin besteht hierdurch die Möglichkeit einer zentralen Überwachung durch die in der zentralen Datenbank hinterlegten Messwerte und Systemparameter. Eine aktive Regelung und Steuerung ist also auch im Gefahrenfall von einem zentralen Ort aus möglich.

Ein Ausführungsbeispiel der Erfindung wird anhand einer Zeichnung näher erläutert. Darin zeigen:
- FIG. 1: eine Biogasanlage mit Reglereinheit, und
- FIG. 2: einen Anlagenverbund mit einer Mehrzahl von Biogasanlagen.

Gleiche Teile sind in allen Figuren mit denselben Bezugszeichen versehen.

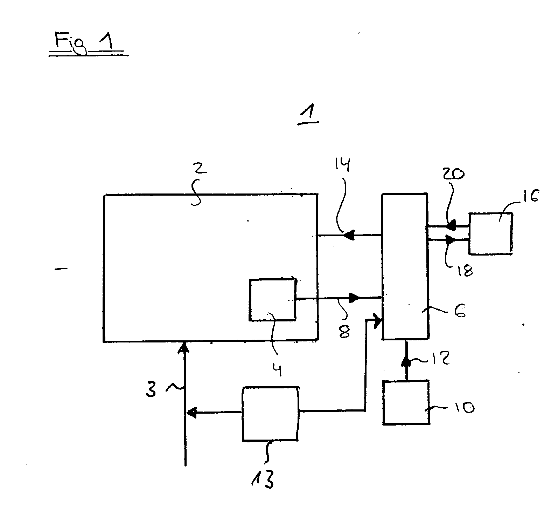

Die Biogasanlage 1 gemäß FIG. 1 umfasst eine Gaserzeugungskammer 2, in der über eine Zuführstrecke 3 Biomasse oder Futter zuführbar ist, aus der im Betriebsfall durch Gärung Biogas unter Ausschluss von Sauerstoff unter Mitwirkung von Bakterien und Enzymen gewonnen wird. Während dieses Prozesses werden durch eine Sensoreneinheit 4 für den Gärprozess charakteristische Messdaten aufgezeichnet. Dies geschieht beispielsweise unter Rückgriff auf eine fortlaufende Analyse der Biomasse. Die Positionierung der Sensorenarbeit ist dabei abhängig von der zu messenden Messgröße und analysiert die gemessenen Messwerte selbstständig und in Echtzeit. Typische Messgrößen sind beispielsweise die Art des entstehenden Gasgemisches, der Wassergehalt der Biomasse, die Temperatur und der entstehende Druck. Diese durch die Sensoreneinheit erfassten Daten werden an eine Reglereinheit 6 übermittelt (dargestellt durch Pfeil 8). Diese Reglereinheit vergleicht die eintreffenden Messwerte bzw. die aus ihr ableitbaren charakteristischen Größen für den Betrieb der Biogasanlage mit einem aus einem Sollwertspeicher 10 übertragenen (dargestellt durch Pfeil 12) Sollwerten.

In Ergänzung zu den Sensoren 4 kann zusätzlich auch in der Art einer Voranalyse eine Erfassung von charakteristischen Daten oder Parametern der der Gaserzeugungskammer 2 zugeführten Biomasse oder des Tierfutters vorgesehen sein. Dabei kann insbesondere ein Analysegerät 13 Proben aus dem Futterstrom entnehmen, um diese dann auf ihre theoretische Wertigkeit oder Gasausbeute zu analysieren. Dieser theoretisch ermittelte Maximalwert kann sodann mit dem entsprechend ermittelten Ist-Wert der Gasausbeute verglichen werden.

Bei Abweichungen der Messwerte von den vorgegebenen Sollwerten oder bei zu geringer Ist-Ausbeute im Vergleich zur theoretisch möglichen Gasausbeute werden zur Regelung der Gasausbeute als Führungsgröße von der Reglereinheit 6 Stellwerte bereitgestellt und diese an die Gaserzeugungskammer 2 übermittelt (dargestellt durch Pfeil 14). Dadurch werden zum einen Störfälle zügig erkannt und die Biogasanlage 1 durch die Reglereinheit 6 evtl. kontrolliert abgeschaltet, zum anderen aber auch die Gasausbeute durch Regulierung beispielsweise der Menge der Biomasse oder der Temperatur erhöht und somit ein effizienterer Betrieb der Biogasanlage 1 ermöglicht. Insbesondere aus Sicherheitsgründen kann dabei auch der Druck in der Kammer überwacht werden.

Zum einen zur Protokollierung, zum anderen aber auch zur weiteren Analyse und Verbesserung und damit Effizienzsteigerung der Biogasanlage werden die Messdaten, der aus den Messwerten ermittelte Ist-Wert für die Gasausbeute und die Systemparameter in einer Datenbank 16 hinterlegt (dargestellt durch Pfeil 18). Sie können ebenfalls durch die Reglereinheit 6 oder durch einen externen Zugriff (nicht dargestellt) zur weiteren Verwendung aus dieser Datenbank 16 abgerufen werden (dargestellt durch Pfeil 20).

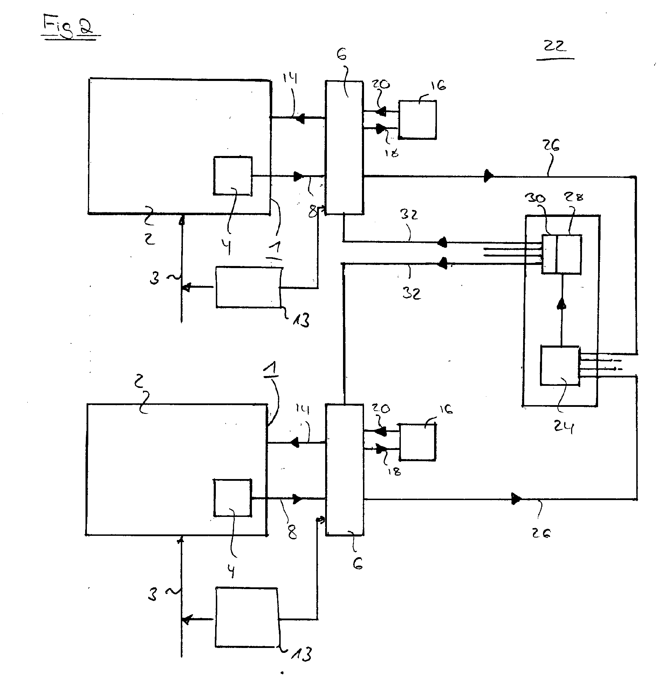

Der Anlagenverbund 22 gemäß FIG. 2 besteht aus einer Mehrzahl von Biogasanlagen 1, wobei in FIG. 2 exemplarisch nur zwei Biogasanlagen dargestellt sind. Jede dieser Biogasanlagen verfügt über eine Gaserzeugungskammer 2, welche eine Sensoreneinheit 4 umfasst, welche Messwerte an eine Reglereinheit 6 überträgt (dargestellt durch Pfeil 8). Diese Messwerte zusammen mit der aus den Messwerten ermittelten Ist-Wert für die Gasausbeute und den Systemparametern können in einer Datenbank 16 hinterlegt werden (dargestellt durch Pfeil 18) und aus ihr für die weitere Verwendung abgerufen werden (dargestellt durch Pfeil 20). Weiterhin werden diese Daten von jeder einzelnen Biogasanlage in einer gemeinsamen zentralen Datenbank 24 hinterlegt (dargestellt durch Pfeil 26). Dies führt dazu, dass diese gemeinsame zentrale Datenbank 24 mit einer Vielzahl von Messgrößen sowie aus ihr abgeleiteten Ist-Werten für die Gasausbeute sowie Systemparametern gefüttert wird. Diese Daten dienen als Input für eine trainierbare Schätzeinheit 28, welche mittels dieser Daten eine optimale Sollwertkonfiguration in Abhängigkeit der festgelegten Systemparameter einer Biogasanlage 1 ermitteln kann.

Durch den Zusammenschluss von mehreren Biogasanlagen und Übertragung der einzelnen Messwerte und Systemparameter in eine gemeinsame zentrale Datenbank 24 kann somit die Gasausbeute und damit die Effizienz jeder einzelnen Biogasanlage 1 erhöht werden. Eine an die trainierbare Schätzeinheit angeschlossene Einstelleinheit 30 übermittelt die von der trainierbaren Schätzeinheit 28 ausgegebene Sollwerte an die Reglereinheit 6 der jeweiligen Biogasanlage 1 (dargestellt durch Pfeil 32). Diese Übertragung der Sollwerte kann beispielsweise von der Biogasanlage 1 aus gefordert werden, beispielsweise bei der erstmaligen Installation oder in regelmäßigen späteren Zyklen. Auf der anderen Seite ist aber auch denkbar, dass in Form von regelmäßigen Updates die Einstelleinheit die einzelnen Biogasanlagen 1 nacheinander mit einem neuen verbesserten Sollwertsatz beliefert. Dies ist durch das stetige Wachsen der gemeinsamen zentralen Datenbank 24 und das dadurch erreichte Beliefern der trainierbaren Schätzeinheit 28 mit neuen Messdatenfamilien möglich.

Grundlage für die trainierbare Schätzeinheit 28 und deren Ausgabe von Soll- oder Stellwerten für die einzelnen Biogasanlagen 1 kann in besonders vorteilhafter Weise die Erfassung der theoretisch durch Probenahmen aus dem Futterstrom ermittelten maximalen Gasausbeute und deren Vergleich mit der tatsächlich erreichten Gasausbeute für eine oder mehrere der Biogasanlagen 1 sein.

Weiterhin ist durch die Übermittlung der Messdaten an eine zentrale Einheit eine stetige zentrale Überwachung der Biogasanlagen zum einen zur Regelung der Effizienz, zum anderen aber auch aus Sicherheitsaspekten möglich. Es ist daher denkbar, dass bei Überschreitung von Toleranzwerten durch die übermittelten Messwerte eine Regelung und Ausgabe von Sollwerten derart geschieht, dass die betreffende Biogasanlage kontrolliert, heruntergefahren oder sogar abgeschaltet wird. Damit ist es für die Überwachung und Steuerung der Biogasanlagen nicht mehr notwendig, für jede Anlage einzeln einen Fachmann vor Ort abzustellen. Durch diese Maßnahme wird ein erheblicher Beitrag zur Rationalisierung der Ressourcen geleistet. Die Wrtschaftlichkeit solcher Biogasanlagen kann hierdurch erheblich erhöht werden.

### Bezugszeichenliste

- 1: Biogasanlage
- 2: Gaserzeugungskammer
- 3: Zuführstrecke
- 4: Sensoreneinheit
- 6: Reglereinheit
- 8: Pfeil
- 10: Sollwertspeicher
- 12: Pfeil
- 13: Analysegerät
- 14: Pfeil
- 16: Datenbank
- 18: Pfeil
- 20: Pfeil
- 22: Anlagenverbund
- 24: zentrale Datenbank
- 26: Pfeil
- 28: trainierbare Schätzeinheit
- 30: Einstelleinheit
- 32: Pfeil

## Patentansprüche

1. Verfahren zum Betreiben einer Biogasanlage (1), bei dem in einer Gaserzeugungskammer (2) eine Anzahl von Messwerten erfasst und überwacht werden, wobei zur Prozessführung eine Anzahl von Stellwerten an die Gaserzeugungskammer (2) übertragen werden und wobei die Stellwerte in Abhängigkeit von vorgegebenen Sollwerten zur Regelung der Gasausbeute als Führungsgröße ermittelt werden.

2. Verfahren nach Anspruch 1, bei dem mindestens ein Sollwert anhand einer durch Analyse der der Gaserzeugungskammer (2) zugeführten Biomasse ermittelten theoretischen Gasausbeute vorgegeben wird.

3. Verfahren nach Anspruch 1 oder 2, bei dem die Messwerte zusammen mit einem für diese Messwerte ermittelten Ist-Wert für die Gasausbeute in einer Datenbank (16) hinterlegt werden.

4. Verfahren nach Anspruch 3, bei dem zusätzlich auch Systemparameter in der Datenbank (16) hinterlegt werden.

5. Verfahren zum Betreiben eines Anlagenverbundes (22) mit einer Mehrzahl von Biogasanlagen (1) nach einem der Ansprüche 1 bis 4, bei dem die Messdaten, der für die Messwerte ermittelte Ist-Wert der Gasausbeute und die Systemparameter in einer gemeinsamen zentralen Datenbank (24) hinterlegt werden.

6. Verfahren nach Anspruch 5, bei dem mittels einer trainierbaren Schätzeinheit (28) aus der gemeinsamen zentralen Datenbank (24) Sollwerte ermittelt werden.

7. Verfahren nach Anspruch 6, bei dem die von der trainierbaren Schätzeinheit (28) ermittelten Sollwerte zur Regelung der Gasausbeute an mindestens eine Biogasanlage (1) übertragen werden.

8. Verfahren nach Anspruch 7, bei dem die Übertragung der Sollwerte durch Abruf der Biogasanlage (1) ausgelöst wird.

9. Verfahren nach einem der Ansprüche 7 oder 8, bei dem die Übertragung der Sollwerte durch Befehl seitens der trainierbaren Schätzeinheit (28) ausgelöst wird.

10. Biogasanlage (1) mit einer Gaserzeugungskammer (2) umfassend eine zur Ermittlung einer Anzahl von Messwerten vorgesehenen Sensoreneinheit (4) und einer Reglereinheit (6), wobei die Reglereinheit (6) in Abhängigkeit der Messwerte und vorgegebener Sollwerte zur Regelung der Gasausbeute ausgelegt ist.

11. Biogasanlage (1) nach Anspruch 10, wobei die Messwerte, der für diese Messwerte ermittelten Ist-Wert der Gasausbeute und/oder die Systemparameter in einer Datenbank (16) hinterlegt sind.

12. Anlagenverbund (22) mit einer Mehrzahl von Biogasanlagen (1) nach einem der Ansprüche 10 oder 11 und einer gemeinsamen zentralen Datenbank (24), wobei die Messwerte, der für diese Messwerte ermittelten Ist-Wert der Gasausbeute und die Systemparameter in dieser gemeinsamen zentralen Datenbank (24) hinterlegt sind.

13. Anlagenverbund (22) nach Anspruch 12, wobei eine gemeinsame, an der zentralen Datenbank (24) angeschlossene und eine trainierbare Schätzeinheit (28) umfassende Einstelleinheit zur Fernregelung mindestens einer Biogasanlage (1) ausgelegt ist.
